# EUROPEAN PATENT SPECIFICATION

(11) **EP 2 409 157 B1**
(45) Date of publication and mention of the grant of the patent: **04.11.2015**
(21) Application number: 10754233.4
(22) Date of filing: 22.03.2010
(51) Int. Cl.: G01N 33/68, G01N 33/53

(54) **CITRULLINE, A RISK INDICATOR FOR CARDIOVASCULAR DISEASE**
CITRULLIN ALS RISIKOINDIKATOR FÜR HERZ-KREISLAUF-ERKRANKUNG
CITRULLINE, INDICATEUR DE RISQUE DE MALADIE CARDIOVASCULAIRE

(30) Priority: 28.05.2009 US 181855 P; 20.03.2009 US 161841 P
(43) Date of publication of application: 25.01.2012
(73) Proprietor: THE CLEVELAND CLINIC FOUNDATION, Cleveland, OH 44195 (US)
(72) Inventor: HANZEN, Stanley, L., Pepper Pike OH 44124 (US); WANG, Zeneng, Shaker Heights Ohio 441212 (US)
(74) Representative: Carlisle, Julie
(86) International application number: PCT/US2010/028156
(87) International publication number: WO 2010/108180

(56) References cited:
- WO-A1-2009/032722
- WO-A2-2006/060793
- WO-A2-2007/103124
- US-A1- 2003 235 573
- US-A1- 2004 048 286
- US-A1- 2007 148 661
- CHO LESLIE ET AL: "Plasma levels of arginine/(ornithine plus citrulline), a global index of functional arginine bioavailability, predicts future risk for major adverse cardiac events", JOURNAL OF THE AMERICAN COLLEGE OF CARDIOLOGY, ELSEVIER, NEW YORK, NY, US, vol. 49, no. 9, Suppl. A, 1 March 2007 (2007-03-01), pages 337A-338A, XP009160744, ISSN: 0735-1097
- NICHOLLS STEPHEN J ET AL: "Metabolic profiling of arginine and nitric oxide pathways predicts hemodynamic abnormalities and mortality in patients with cardiogenic shock after acute myocardial infarction", CIRCULATION, vol. 116, no. 20, November 2007 (2007-11), pages 2315-2324, XP002679322, ISSN: 0009-7322
- GARLICHS C D ET AL: "DECREASED PLASMA CONCENTRATIONS OF L-HYDROXY-ARGININE AS A MARKER OF REDUCED NO FORMATION IN PATIENTS WITH COMBINED CARDIOVASCULAR RISK FACTORS", JOURNAL OF LABORATORY AND CLINICAL MEDICINE, MOSBY, INC, US, vol. 135, no. 5, 1 May 2000 (2000-05-01), pages 419-425, XP008004793, ISSN: 0022-2143, DOI: 10.1067/MLC.2000.105975
- ROMERO MARITZA J ET AL: "Therapeutic use of citrulline in cardiovascular disease", CARDIOVASCULAR DRUG REVIEWS, NEVA PRESS, BRANFORD, CT, US, vol. 24, no. 3-4, 1 October 2006 (2006-10-01), pages 275-290, XP002526178, ISSN: 0897-5957, DOI: 10.1111/J.1527-3466.2006.00275.X
- TANG W H W ET AL: "Diminished Global Arginine Bioavailability and Increased Arginine Catabolism as Metabolic Profile of Increased Cardiovascular Risk", JOURNAL OF THE AMERICAN COLLEGE OF CARDIOLOGY, ELSEVIER, NEW YORK, NY, US, vol. 53, no. 22, 2 June 2009 (2009-06-02), pages 2061-2067, XP026140026, ISSN: 0735-1097, DOI: 10.1016/J.JACC.2009.02.036 [retrieved on 2009-05-26]
- PENTTINEN J ET AL: "Indicators of L-arginine metabolism and cardiovascular risk factors--a cross-sectional study in healthy middle-aged men.", AMINO ACIDS 2000 LNKD- PUBMED:10901617, vol. 18, no. 3, 2000, pages 199-206, XP002679323, ISSN: 0939-4451
- SMITH ET AL: "Nitric oxide precursors and congenital heart surgery: A randomized controlled trial of oral citrulline", JOURNAL OF THORACIC AND CARDIOVASCULAR SURGERY, MOSBY-YEAR BOOK, INC., ST. LOUIS, MO, US, vol. 132, no. 1, 1 July 2006 (2006-07-01), pages 58-65, XP005693517, ISSN: 0022-5223, DOI: 10.1016/J.JTCVS.2006.02.012
- MARTENS-LOBENHOFFER J ET AL: "Simultaneous detection of arginine, asymmetric dimethylarginine, symmetric dimethylarginine and citrulline in human plasma and urine applying liquid chromatography-mass spectrometry with very straightforward sample preparation", JOURNAL OF CHROMATOGRAPHY B: BIOMEDICAL SCIENCES & APPLICATIONS, ELSEVIER, AMSTERDAM, NL, vol. 798, no. 2, 25 December 2003 (2003-12-25), pages 231-239, XP004474594, ISSN: 1570-0232, DOI: 10.1016/J.JCHROMB.2003.09.050
- PAPADIA C ET AL: "PLASMA CITRULLINE AS A QUANTITATIVE BIOMARKER OF HIV-ASSOCIATED DUODENAL MUCOSAL DAMAGE IN A TROPICAL ENTEROPATHY POPULATION", DIGESTIVE AND LIVER DISEASE, W.B. SAUNDERS, GB, vol. 41, 1 March 2009 (2009-03-01), pages S65-S66, XP026191208, ISSN: 1590-8658, DOI: 10.1016/S1590-8658(09)60170-5 [retrieved on 2009-03-01]
- "624 Poster A linear dose response relationship at a clinically relevant dose range exists for plasma citrulline levels after single dose whole body irradiation in mice", RADIOTHERAPY AND ONCOLOGY, ELSEVIER, IRELAND, vol. 64, 1 September 2002 (2002-09-01), page S192, XP005057471, ISSN: 0167-8140
- MORRIS CLAUDIA R ET AL: "Dysregulated arginine metabolism, hemolysis-associated pulmonary hypertension, and mortality in sickle cell disease", JAMA THE JOURNAL OF THE AMERICAN MEDICAL ASSOCIATION, AMERICAN MEDICAL ASSOCIATION, US, vol. 294, no. 1, 1 July 2005 (2005-07-01), pages 81-90, XP002504905, ISSN: 0098-7484, DOI: 10.1001/JAMA.294.1.81
- PENTTINEN J. ET AL.: 'Indicators of L-arginine metabolism and cardiovascular risk factors--a cross-sectional study in healthy middle-aged men' AMINO AC IDS vol. 18, no. 3, 2000, pages 199 - 206, XP002679323

## Description

### RELATED APPLICATIONS

The present application claims priority from provisional application serial number 61/161,841, entitled "Citrulline, A Risk Indicator for Cardiovascular Disease," filed on March 20, 2009, and provisional patent application serial number 61/181,855, entitled "Citrulline for Diagnosis and Prediction of Heart Failure and Aortic Disorders," filed on May 28, 2009.

### GOVERNMENT RIGHTS

The work described in this application was supported, at least in part, by Grant No. P01 HL076491-055328, P01 HL087018-020001, and P50 HL077107-050004 from the National Institutes of Health. The United States government has certain rights in this invention

### BACKGROUND

Cardiovascular disease (CVD) accounts for one in every two deaths in the United States and is the number one killer disease in the United States and most European Countries. Thus, the diagnosis and prevention of cardiovascular disease are areas of major public health importance. A low-fat diet and exercise are recommended to prevent CVD. In addition, a number of therapeutic agents may be prescribed by medical professionals to those individuals who are known to be at risk of having CVD. More aggressive therapy, such as administration of multiple medications or surgical intervention may be used in those individuals who are at high risk of having CVD. Since CVD therapies may have adverse side effects, it is desirable to have methods for identifying those individuals who are at risk, particularly those individuals who are at high risk of experiencing an adverse cardiovascular event near term.

Currently, several risk factors are used by medical professionals to assess an individual's risk of developing or having CVD and to identify individuals at high risk. Major risk factors for cardiovascular disease include age, hypertension, family history of premature CVD, smoking, high total cholesterol, low HDL cholesterol, obesity and diabetes. The major risk factors for CVD are additive, and are typically used together by physicians in a risk prediction algorithm to target those individuals who are most likely to benefit from treatment for CVD. These algorithms achieve a high sensitivity and specificity for predicting risk of CVD within 10 years. However, the ability of the present algorithms to predict a higher probability of developing CVD is limited. Among those individuals with none of the current risk factors, the 10-year risk for developing CVD is still about 2%. In addition, a large number of CVD complications occur in individuals with apparently low to moderate risk profiles, as determined using currently known risk factors. Thus, there is a need to expand the present cardiovascular risk algorithm to identify a larger spectrum of individuals at risk for or affected with CVD.

A significant factor in the development of cardiovascular disease is the presence of atherosclerosis. However, the mechanism of atherosclerosis is not well understood. Over the past decade a wealth of clinical, pathological, biochemical and genetic data support the notion that atherosclerosis is a chronic inflammatory disorder. Acute phase reactants (e.g. C-reactive protein, complement proteins), sensitive but non-specific markers of inflammation, are enriched in fatty streaks and later stages of atherosclerotic lesions. In a recent prospective clinical trial, base-line plasma levels of C-reactive protein independently predicted risk of first-time myocardial infarction and stroke in apparently healthy individuals. U.S. Pat. No. 6,040,147 describes methods which use C-reactive protein, cytokines, and cellular adhesion molecules to characterize an individual's risk of developing a cardiovascular disorder. Although useful, these markers may be found in the blood of individuals with inflammation due to causes other than CVD, and thus, these markers may not be specific enough. Moreover, modulation of their levels has not been shown to predict a decrease in the morbidity or mortality of CVD. Accordingly, there exists a need for additional markers for assessing a subject's risk of having or developing CVD.

### SUMMARY OF THE INVENTION

The present invention provides new diagnostic tests and methods for characterizing an individual's risk of having or developing cardiovascular disease (CVD), monitoring an individual's response to intervention aimed at preventing or reducing CVD, assessing an individual's risk of experiencing an adverse cardiovascular event, and/or determining whether a subject requires intervention as a result of being at risk of having CVD. The described methods are useful for identifying those individuals who are in need of intervention aimed at reducing the risk or severity of CVD as well as those individuals who do not require aggressive intervention targeted at preventing CVD.

The methods described herein are based on the discovery that patients with cardiovascular disease have significantly greater levels of free citrulline than patients without significant CVD. It has also been discovered that the relationship between higher citrulline levels and prevalence of CVD is independent of traditional CVD risk factors, such as Framingham Global Risk Score, C-reactive protein levels, and renal function.

Thus, the present diagnostic methods, which involve assessing levels of free citrulline in a sample of urine, blood, serum, or plasma obtained from a subject, provide additive predictive value beyond that seen with clinical and diagnostic risk factors currently employed by physicians.

Accordingly, in one aspect the present invention provides a method for characterizing a human subject's risk of having cardiovascular disease (CVD) that includes determining a level of free citrulline in a bodily sample of urine, blood, serum, or plasma, from the subject; comparing the subject's level of free citrulline with a control value based on levels of free citrulline in comparable bodily samples from a control population; and characterizing the subject's risk of having CVD based on said comparison; in which the subject is characterized as being at risk of having CVD if the subject's level of free citrulline is greater than the control value.

In another aspect, the present invention provides a method for characterizing a human subject's risk of developing cardiovascular disease (CVD) that includes:determining a level of free citrulline in a bodily sample of urine, blood, serum, or plasma, from the subject; comparing the subject's level of free citrulline with a control value based on levels of free citrulline in comparable bodily samples from a control population; and characterizing the subject's risk of developing CVD based on said comparison; in which the subject is characterized as being at risk of developing CVD if the subject's level of free citrulline is greater than the control value.

In another aspect, the present invention provides a method for monitoring a human subject's response to intervention aimed at reducing risk of CVD, including determining a first and second level of free citrulline in a bodily sample of urine, blood, serum, or plasma, from the subject before and after administration of the intervention, respectively; comparing the subject's first and second levels of free citrulline; and characterizing the subject's response to the intervention based on said comparison; in which the subject is characterized as responding to the intervention if the subject's second level of free citrulline is lesser than the first level of free citrulline.

In any of these aspects, the subject's level of free citrulline can be used to provide a risk predictor that is independent of arginine bioavailability ratios. In some embodiments, the cardiovascular disease is heart failure, whereas in others it can be aortic dissection, aortic aneurysm, or a major adverse cardiac event.

In any of these aspects, the control value can be a single representative value or a range of representative values and be based upon the levels of free citrulline in comparable bodily samples from the general population or a select population of human subjects. Alternately, the control value includes ranges divided into a plurality of groups, and said comparing step comprises determining into which group the subject's level of said risk predictor falls. If a plurality of groups are used, they can include at least a high risk range and a low risk range.

All aspects can also include administering or prescribing an intervention targeted at reducing the risk of CVD to a subject who has been characterized as being at risk of having CVD, which can be administration of a therapeutic agent.

In all the aspects described herein, the subject's level of free citrulline may be determined using a number of techniques, including: immunological techniques, mass spectrometry, high performance liquid chromatography (HPLC), or any combination thereof.

### BRIEF DESCRIPTION OF THE FIGURES

Figure 1 is a schematic illustration of pathways for nitric oxide production and arginine catabolism. Caption: CPS = carbamyl phosphate synthetase; ADMA = asymmetric dimethylarginine; L-NMMA = N(G)-mono-methyl-L-arginine.
Figure 2 provides two bar graphs showing the distribution of myocardial infarction or stroke, all-cause mortality, and 3-year incidence of major adverse cardiac events (MACE) according to the global arginine bioavailability ratio (GABR; upper graph) and arginine quartiles (lower graph). P = P-value for trend.
Figure 3 is a graph showing the Kaplan-Meier survival analysis for patients with 3-year incidence of major adverse cardiac events (%MACE) according to global arginine bioavailability ratio (GABR) quartiles.
Figure 4 is a graph showing the Kaplan-Meier survival analysis for patients with 3-year incidence of major adverse cardiac events (%MACE) according to citrulline quartiles.
Figure 5 provides two graphs showing a distribution analysis of citrulline levels in subjects. Section 5A compares the citrulline levels between subjects with chronic heart failure and acute heart failure, while section 5B compares the citrulline levels between control subjects and subjects with aortic dissection.

### DETAILED DESCRIPTION OF THE INVENTION

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described.

As used herein and in the appended claims, the singular forms "a", "and", and "the" include plural referents unless the context clearly dictates otherwise. Thus, for example, reference to "a sample" includes a plurality of such samples, and so forth.

### DEFINITIONS

The terms "individual," "host," "subject," and "patient" are used interchangeably herein, and generally refer to a mammal, including, but not limited to, primates, including simians and humans, equines (*e.g*., horses), canines (*e.g.*, dogs), felines, various domesticated livestock (*e.g*., ungulates, such as swine, pigs, goats, sheep, and the like), as well as domesticated pets and animals maintained in zoos. In some embodiments, the subject is specifically a human subject.

As used herein, the terms "cardiovascular disease" (CVD) or "cardiovascular disorder" are terms used to classify numerous conditions affecting the heart, heart valves, and vasculature (e.g., veins and arteries) of the body and encompasses diseases and conditions including, but not limited to arteriosclerosis, atherosclerosis, myocardial infarction, acute coronary syndrome, angina, congestive heart failure, aortic aneurysm, aortic dissection, iliac or femoral aneurysm, pulmonary embolism, primary hypertension, atrial fibrillation, stroke, transient ischemic attack, systolic dysfunction, diastolic dysfunction, myocarditis, atrial tachycardia, ventricular fibrillation, endocarditis, peripheral vascular disease, coronary artery disease (CAD), peripheral artery disease (PAD), and cerebrovascular disease.

As used herein, the term "atherosclerotic cardiovascular disease" or "disorder" refers to a subset of cardiovascular disease that include atherosclerosis as a component or precursor to the particular type of cardiovascular disease and includes, without limitation, CAD, PAD, cerebrovascular disease. Atherosclerosis is a chronic inflammatory response that occurs in the walls of arterial blood vessels. It involves the formation of atheromatous plaques that can lead to narrowing ("stenosis") of the artery, and can eventually lead to partial or complete closure of the arterial opening and/or plaque ruptures. Thus atherosclerotic diseases or disorders include the consequences of atheromatous plaque formation and rupture including, without limitation, stenosis or narrowing of arteries, heart failure, aneurysm formation including aortic aneurysm, aortic dissection, and ischemic events such as myocardial infarction and stroke

A cardiovascular event, as used herein, refers to the manifestation of an adverse condition in a subject brought on by cardiovascular disease, such as sudden cardiac death or acute coronary syndromes including, but not limited to, myocardial infarction, unstable angina, aneurysm, or stroke. The term "cardiovascular event" can be used interchangeably herein with the term cardiovascular complication. While a cardiovascular event can be an acute condition, it can also represent the worsening of a previously detected condition to a point where it represents a significant threat to the health of the subject, such as the enlargement of a previously known aneurysm or the increase of hypertension to life threatening levels. A major cardiovascular event is a non-fatal myocardial infarction, stroke, death, or a combination thereof.

As used herein, the term "diagnosis" can encompass determining the nature of disease in a subject, as well as determining the severity and probable outcome of disease or episode of disease and/or prospect of recovery (prognosis). "Diagnosis" can also encompass diagnosis in the context of rational therapy, in which the diagnosis guides therapy, including initial selection of therapy, modification of therapy (*e.g.,* adjustment of dose and/or dosage regimen), and the like.

The term "intervention" or "therapy" targeted at CVD, or targeted at preventing or reducing CVD, and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Therapy" as used herein, covers any treatment of a disease in a subject, and can include: (a) preventing the disease or a symptom of a disease from occurring in a subject which may be predisposed to the disease but has not yet been diagnosed as having it; (b) inhibiting or retarding the disease or condition, *i.e*., arresting or slowing its development or progression; and (c) relieving the disease, *i.e*., causing regression of the disease.

The term "intervention" as used herein includes one or more therapeutic agents targeted at CVD, (*e.g*. anti-inflammatory drugs, cholesterol lowering drugs, etc.), as well as life style changes (*e.g*. change in diet, exercise regime, weight loss, etc.).

"Arginine" or "Arg" or "L-Arg" as used herein refers to naturally occurring L-arginine. Free citrulline refers to citrulline that is not peptide bound.

The methods of the present invention can be directed to the detection, monitoring, or diagnosis of subjects with regard to specific cardiovascular diseases or cardiovascular events. For example, the methods of the invention can be directed to identifying subjects at risk of developing heart failure or aortic disorders such as aortic aneurysm or aortic dissection.

Heart failure is a form of cardiovascular disease is a condition in which a problem with the structure or function of the heart impairs its ability to supply sufficient blood flow to meet the body's needs, characterized by compromised ventricular systolic or diastolic functions, or both. Heart failure may be manifested by symptoms of poor tissue perfusion alone (*e.g*., fatigue, poor exercise tolerance, and/or confusion) or by both symptoms of poor tissue perfusion and congestion of vascular beds (*e.g*., dyspnea, chest rates, pleural effusion, pulmonary edema, distended neck veins, congested liver, and/or peripheral edema). Congestive heart failure represents a form of heart failure where cardiac output is low, in contrast with high output cardiac failure, in which the body's requirements for oxygen and nutrients are increased, and demand outstrips what the heart can provide.

Heart failure can occur as a result of one or more causes. A major cause is secondary atherosclerotic disease, where one or more ischemic events such as a heart attack result in ischemic injury to the heart and decreased function. This type of heart failure is referred to as ischemic heart failure, because the cause of the cardiac dysfunction was secondary to the ischemic injury. Ischemic heart failure can also result from other cardiovascular conditions leading to ischemic injury, such as atherosclerosis that limits blood flow.

Heart failure can also occur as a result of causes other than ischemia, and such forms of heart failure are referred to as non-ischemic heart failure. Examples of non-ischemic heart failure include myocarditis resulting from viral infection, amyloidosis of cardiac tissue, arrhythmia, manifestation of genetic defects, injury from abuse of alcohol, drugs, or cigarettes, other sources of injury to cardiac tissue such as infection by bacteria or parasites, or vitamin deficiency.

Aortic dissection is a tear in the wall of the aorta that causes blood to flow between the layers of the wall of the aorta and force the layers apart. In an aortic dissection, blood penetrates the intima, which is the innermost layer of the aortic artery, and enters the media layer. The high pressure rips the tissue of the media apart along the laminated plane splitting the inner 2/3 and the outer 1/3 of the media apart. This can propagate along the length of the aorta for a variable distance forward or backwards. Dissections that propagate towards the iliac bifurcation (with the flow of blood) are called anterograde dissections and those that propagate towards the aortic root (opposite of the flow of blood) are called retrograde dissections. The initial tear is usually within 100 mm of the aortic valve so a retrograde dissection can easily compromise the pericardium leading to a hemocardium. Aortic dissection is a medical emergency and can quickly lead to death, even with optimal treatment.

Symptoms of aortic dissection are known to those skilled in the art, and include severe pain that had a sudden onset that may be described as tearing in nature, or stabbing or sharp in character. Some individuals will report that the pain migrates as the dissection extends down the aorta. While the pain may be confused with the pain of a myocardial infarction, aortic dissection is usually not associated with the other signs that suggest myocardial infarction, including heart failure, and ECG changes. Individuals experiencing an aortic dissection usually do not present with diaphoresis (profuse sweating). Individuals with chronic dissection may not indicate the presence of pain. Aortic insufficiency is also typically seen. Other less common symptoms that may be seen in the setting of aortic dissection include congestive heart failure (7%), syncope (9%), cerebrovascular accident (3-6%), ischemic peripheral neuropathy, paraplegia, cardiac arrest, and sudden death. Preferably, this diagnosis is made by visualization of the intimal flap on a diagnostic imaging test such as a CT scan of the chest with iodinated contrast material and a trans-esophageal echocardiogram.

An aortic aneurysm, on the other hand, is a cardiovascular disorder characterized by a swelling of the aorta, which is usually caused by an underlying weakness in the wall of the aorta at that location. Aortic aneurysms are classified by where they occur on the aorta. Abdominal aortic aneurysms, hereafter referred to as AAAs, are the most common type of aortic aneurysm, and are generally asymptomatic before rupture. The most common sign for the aortic aneurysm is the Erydema Nodosum also known as leg lesions typically found near the ankle area. AAAs are attributed primarily to atherosclerosis, though other factors are involved in their formation. An AAA may remain asymptomatic indefinitely. There is a large risk of rupture once the size has reached 5 cm, though some AAAs may swell to over 15 cm in diameter before rupturing. Only 10-25% of patients survive rupture due to large pre- and post-operative mortality.

Symptoms of an aortic aneurysm may include: anxiety or feeling of stress; nausea and vomiting; clammy skin; rapid heart rate. However, an intact aortic aneurysm may not produce symptoms. As they enlarge, symptoms such as abdominal pain and back pain can develop. Compression of nerve roots may cause leg pain or numbness. Untreated, aneurysms tend to become progressively larger, although the rate of enlargement is unpredictable for a given individual. In some cases, clotted blood which lines most aortic aneurysms can break off and result in an embolus. Preferably, medical imaging is used to confirm the diagnosis of an aortic aneurysm.

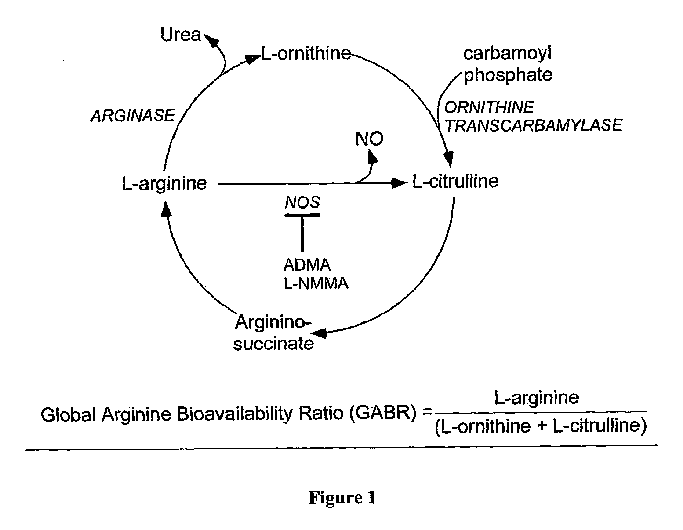

The term "arginine bioavailability," as used herein, refers to the ratio of arginine levels divided by modulators of arginine bioavailability, including arginine metabolites. "Modulators of arginine availability" are compounds that affect the metabolism of arginine. Such modulators include substrates or products of an arginine metabolism pathway (*e.g*., nitric oxide synthase (NOS), arginase, and the like), as well as compounds that promote or inhibit activity of an amino acid transporter or enzyme involved in an arginine metabolism pathway (*e.g*., NOS, arginase, and the like; see Figure 1 for review of arginine pathway involving NOS and arginase). Exemplary modulators include amino acids and metabolites of amino acids that are metabolites of arginine or are metabolized to arginine metabolites from enzymes that directly produce or utilize arginine, including but not limited to NOS and arginase, or metabolites that result from metabolism of citrulline to arginine in the kidney (which is reduced under condition of renal insufficiency or dysfunction/injury), and amino acid metabolites that impact transport of arginine or represent arginine analogues (methylated arginines) thereby impacting arginine bioavailability. The term "arginine metabolite" refers to a product of action of nitric oxide synthase (NOS) or arginase on arginine, as well as metabolites from arginine:glycine amidinotransferase and arginine decarboxylase activity on arginine. "Arginine metabolite" can include immediate metabolites of NOS , arginase or other arginine catabolizing enzymes' action upon arginine (*e.g*., citrulline, ornithine, creatine and the like) or downstream products of such metabolites (*e.g.* proline, polyamines and the like. The calculation of the final ratio of arginine to such modulators or metabolites are reflective of arginine bioavailability. Accordingly, the term Global Arginine Bioavailability Ratio or GABR, as used in the Example, refers to the ratio arginine / (ornithine + citrulline). Arginine bioavailability is defined in Morris & Hazen's US Application No: 11/293,351, filed 12/01/2005.

The term "near-term" refers to within 3 years. Accordingly, a "near-term risk of experiencing a major adverse cardiovascular event" refers to the risk of experiencing such an event in the following 3 months, 6 months, 1 year or 3 years.

The term "long-term" refers to more than 3 years. Accordingly, a "long-term risk of experiencing a major adverse cardiovascular event" refers to the risk of experiencing such an event in the following 5 or 10 years.

The present invention provides new tests and methods for characterizing an individual's risk of having or developing cardiovascular disease (CVD), monitoring an individual's response to intervention aimed at preventing or reducing CVD, assessing an individual's risk of experiencing an adverse cardiovascular event, and/or determining whether a subject requires intervention as a result of being at risk of having CVD. The described methods are useful for identifying those individuals who are in need of intervention aimed at reducing the risk or severity of CVD as well as those individuals who do not require aggressive intervention targeted at preventing CVD.

The methods include determining the level of free citrulline in a bodily sample, *e.g.,* of urine, blood, plasma or serum, obtained from the subject.

The present invention also relates to kits that comprise assays for free citrulline levels. Such assays have appropriate sensitivity with respect to control values selected on the basis of the present tests. The present kits can further include, for example, cut-off values, sensitivities at particular cut-offs, as well as instructions or other printed material for characterizing risk based upon the outcome of the assay.

### Preparation of Bodily Sample

Whole blood is obtained from the individual or test subject using standard clinical procedures. Plasma is obtained from whole blood samples by centrifugation of anti-coagulated blood. Such process provides a buffy coat of white cell components and a supernatant of the plasma.

Serum is collected by centrifugation of whole blood samples that have been collected in tubes that are free of anti-coagulant. The blood is permitted to clot prior to centrifugation. The yellowish-reddish fluid that is obtained by centrifugation is the serum.

The subject's tested in the methods described herein may already have CVD, may be suspected to be at risk of having CVD, or may be those who would not traditionally be considered at risk of developing CVD, such as non-diabetic, non-hypertensive, and non-smokers.

In other embodiments, the subject already exhibits symptoms of cardiovascular disease. For example, the subjects may exhibit symptoms of heart failure or an aortic disorder such as aortic dissection or aortic aneurysm. For subjects already experiencing cardiovascular disease, the levels of citrulline may be used to predict the likelihood of further cardiovascular events or the outcome of ongoing cardiovascular disease.

In certain embodiments the test subject is a smoker. In certain embodiments, the test subject is a non-smoker. In certain embodiments, the test subject has normal renal function. In certain embodiments, the test subject does not have normal renal function, *e.g*., the subject has chronic kidney disease. In certain embodiments, the test subject is a diabetic. In certain embodiments, the test subject is non-diabetic. In certain embodiments, the control subjects are members of the general population. In certain embodiments, the test subject and/or control subjects lacks symptoms of cardiovascular disease. In certain embodiments, the test subject and/or control subjects lack clinical evidence of cardiovascular disease. In certain embodiments, the control subjects are matched to the test subject, *e.g*. the test subject and control subjects are in the same age bracket, are the same gender, etc..

### Methods of Determining Free Citrulline levels

Citrulline levels may be determined by any of a variety of standard methods known in the art.

One method includes using immunological assays, including enzyme-linked immunosorbent assay (ELISA), radioimmunoassay (RIA), etc. Immunological assays include sandwich-type assays, competitive assays, etc. Immunological assays generally involve use of an antibody specific for citrulline where the antibody is detectably labeled, either directly or indirectly. Suitable direct labels include, but are not limited to, radioactive labels (*e.g.*, I¹²⁹, etc.); enzyme labels, where the enzyme generates a product that is detectable by a colorimetric or fluorimetric assay, e.g., p-galactosidase, luciferase, horse radish peroxidase, alkaline phosphatase; fluorescent proteins, e.g. a green fluorescent protein; and the like. Indirect labels include secondary antibodies that are detectably labeled; a member of a specific binding pair (e.g., biotin/avidin, etc.) that is detectably labeled; and the like.

Other methods include high performance liquid chromatography (HPLC), including reverse phase HPLC.

Other suitable methods include the use of mass spectrometry, spectrophotometric methods, tandem mass spectrometry methods, etc. Suitable methods for determining a level of citrulline have been reported in the literature. See, *e.g.,* U.S. Patent No. 6,720,188; Teerlink et al. (2002) Anal. Biochem. 303:131-137; Dobashi et al. (2002) Analyst 127:54-59; Pi et al. (2000) J. Chromatogr. B. Biomed. Sci. Appl. 742:199-203; Chen et al. (1997) J. Chromatogr. B. Biomed. Sci. Appl. 692:467-471; Anderstam et al. (1997) J. Am. Soc. Nephrol. 8:1487-1442; Pettersson et al. (1997) J. Chromatogr. B. Biomed. Sci. Appl. 692:257-262; Sultana et al. (2001) J. Chromatogr. B. Biomed. Sci. Appln. 755:321; Chace et al. (2003) Clin. Chem. 49: 1797-1817; and Trapp et al. (2004) J. Sep. Sci. 27: 1483-1490.

### Control Value

The level of citrulline in the bodily sample obtained from the subject is compared to a control value obtained from a reference cohort. In some embodiments, the reference cohort is the general population. In certain embodiments, the reference cohort is a select population of human subjects.

In certain embodiments, the reference cohort is comprised of individuals who have not previously had any signs or symptoms indicating the presence of atherosclerosis, such as angina pectoris, history of an acute adverse cardiovascular event such as a myocardial infarction or stroke, evidence of atherosclerosis by diagnostic imaging methods including, but not limited to coronary angiography. In certain embodiments, the reference cohort is comprised of individuals, who if examined by a medical professional would be characterized as free of symptoms of disease. In another example, the reference cohort may be individuals who are nonsmokers. "Nonsmoker", as used herein, means an individual who, at the time of the evaluation, is not a smoker and has not used a tobacco product for the preceding 1 year period. This includes individuals who have never smoked as well as individuals who in the past have smoked but has not smoked for the past year. A nonsmoker cohort may have a different normal level of free citrulline than will a smoking population or the general population. Accordingly, the control values selected may take into account the category into which the test subject falls.

In other embodiments, the reference cohort is comprised of individuals with renal dysfunction or impairment. Renal function can be assessed by standard methods, for example by determining the subject serum creatinine levels or blood urea nitrogen levels. Accordingly, subjects with renal dysfunction or impairment do not have normal renal function, e.g. suffer from chronic kidney disease.

Appropriate categories can be selected with no more than routine experimentation by those of ordinary skill in the art. As further information becomes available as a result of routine performance of the methods described herein, population average values for free citrulline levels may be used.

In other embodiments, "normal" citrulline may be obtained by determining the free citrulline levels in samples obtained from subjects without CVD, subjects who do not develop CVD in a prescribed period of time, from archived patient samples, and the like.

The control value is related to the value used to characterize the level of citrulline in the bodily sample obtained from the subject. Thus, if the level of free citrulline is an absolute value, such as the units of free citrulline per milliliter of blood, plasma, serum, or urine, the control value is also based upon the units of free citrulline per milliliter of a comparable bodily sample (*i.e.* blood, plasma, serum, or urine) in individuals in the general population or a select population of human subjects. Similarly, if the free citrulline level is a representative value such as an arbitrary unit, the control value is also based on the representative value.

The control value can take a variety of forms. The control value can be a single cut-off value, such as a median or mean. The control value can be established based upon comparative groups such as where the risk in one defined group is double the risk in another defined group. The control values can be divided equally (or unequally) into groups, such as a low risk group, a medium risk group, and a high-risk group, or into quadrants, the lowest quadrant being individuals with the lowest risk the highest quadrant being individuals with the highest risk, and the test subject's risk of having CVD can be based upon which group his or her test value falls. For example, the low risk group can represent a below average risk, the medium risk group can represent an average risk, and the high risk group can represent an above average risk. High risk groups can also include individuals with additional risk factors, such as hypertension or smoking, whereas low risk groups can include individuals without any additional risk factors.

Control values of free citrulline in biological samples obtained, such as for example, mean levels, median levels, or "cut-off' levels, are established by assaying a large sample of individuals in the general population or the select population and using a statistical model such as the predictive value method for selecting a positivity criterion or receiver operator characteristic curve that defines optimum specificity (highest true negative rate) and sensitivity (highest true positive rate) as described in Knapp, R.G., and Miller, M.C. (1992), Clinical Epidemiology and Biostatistics, William and Wilkins, Harual Publishing Co. Malvern, PA. A "cutoff" value can be determined for each risk marker that is assayed

### Comparison of Citrulline Levels in the Subject to the Control Value

The levels of the citrulline-based risk predictor in the individual's bodily sample may be compared to a single control value or to a range of control values, as described above. If the level of citrulline in the subject is greater than the control value or is in the upper range of control values, the subject is at greater risk of developing or having CVD than individuals with levels comparable to or below the control value or in the lower range of control values. In contrast, if the level of citrulline in the subject is below the control value or is in the lower range of control values, the subject is at a lower risk of developing or having CVD than individuals with levels comparable to or above the control value or exceeding or in the upper range of control values.

The extent of the difference between the subject's citrulline levels and control value is also useful for characterizing the extent of the risk and thereby, determining which individuals would most greatly benefit from certain interventions. In those cases where the control value ranges are divided into a plurality of groups, such as the control value ranges for individuals at high risk, average risk, and low risk, the comparison involves determining into which group the test subject's level of the relevant risk predictor falls.

Alternatively, the level of free citrulline may be compared to the level of an internal standard in the sample. Examples of suitable internal standards include, but are not limited to, levels of oxidized total protein in the subject's bodily sample, levels of oxidized albumin in the subject's bodily sample, unoxidized PON1, apolipoprotein A-1 (apoA1), HDL particle number, and/or HDL cholesterol levels.

The present methods are useful for determining if and when interventions (including therapeutic agents or life-style changes) which are targeted at preventing or reducing CVD or for slowing the progression of CVD should and should not be prescribed for an individual. For example, individuals with citrulline risk predictor values above a certain cutoff value, or that are in the higher tertile or quartile of a "normal range," could be identified as those in need of more aggressive intervention with lipid lowering agents, and/or life style changes, etc.

An attractive feature of the use of increased citrulline levels as a predictor of risk for CVD is that it represents an independent marker to identify individuals with increased risk for cardiovascular disease and/or for experiencing a major adverse cardiovascular event. That is, in multivariate analyses vs. other known risk factors for CVD (*e.g*. lipid levels such as LDL, HDL, total cholesterol, triglycerides, family history, tobacco use, hypertension, and diabetes, as well as other arginine-related risk predictors such as GABR levels), elevated levels of citrulline independently predicted association with CVD and predicted risk of experiencing a major adverse cardiovascular event. Thus, the present diagnostic tests are especially useful to identify individuals at increased risk who might otherwise not have been identified by existing screening protocols/methods. Moreover, the present risk predictors can be used in combination with currently used risk predictors, such as blood LDL levels, blood triglyceride levels and blood C-reactive protein levels, as well as GABR values, and algorithms based thereon to more accurately characterize an individual's risk of developing or having CVD, or an individual's risk of experiencing a major adverse cardiovascular event.

### Evaluation of CVD Therapeutic Agents

The present methods are also useful for evaluating the subject's response to interventions targeted at preventing or reducing CVE, especially in patients who have already been diagnosed as having or as being at risk of developing CVD. Such interventions include the use of therapeutic agents such as, but not limited to, anti-inflammatory agents, insulin sensitizing agents, antihypertensive agents, anti-thrombotic agents, anti-platelet agents, fibrinolytic agents, lipid reducing agents, direct thrombin inhibitors, ACAT inhibitor, CDTP inhibitor thioglytizone, and glycoprotein II b/IIIa receptor inhibitors. Such evaluation comprises determining the subject's citrulline levels in a bodily sample taken from the subject prior to administration of the therapeutic agent and a corresponding bodily sample taken from the subject during or following administration of the therapeutic agent. A decrease in the citrulline levels in the sample taken after or during administration of the therapeutic as compared to the citrulline levels in the sample taken before administration of the therapeutic agent is indicative of a positive effect of the therapeutic agent on cardiovascular disease in the treated subject.

### EXAMPLES

The following examples are for purposes of illustration only and are not intended to limit the scope of the claims.

### EXAMPLE 1

It was hypothesized that an integrated assessment of arginine with its catabolic products may better predict cardiovascular risks than arginine levels alone. Arginine is the sole nitrogen source for nitric oxide (NO) synthesis. The major catabolic products of arginine are ornithine and citrulline. Plasma levels of free arginine, ornithine, citrulline and the endogenous NO synthase inhibitor asymmetric dimethylarginine (ADMA) were measured using LC/MS/MS. The relationship of global arginine bioavailability ratio (GABR, defined as arginine/[ornithine + citrulline]) vs. arginine and its catabolic metabolites to prevalence of coronary artery disease (CAD) and incidence of major adverse cardiovascular events (MACE = death, myocardial infarction, stroke) was examined over a 3-year follow-up in 1,010 subjects undergoing elective cardiac catheterization.

Patients with CAD had significantly lower GABR [median(IQR); 1.06(0.75, 1.31) versus 1.27(0.96, 1.73), p<0.001] and arginine levels [mean: 68 ±20 µM versus 74 ±24 µM, p<0.001) than those without CAD. After adjusting for Framingham risk score, C-reactive protein, and renal function, lower GABR (but not arginine levels) and higher citrulline levels remained significantly associated with both prevalence of CAD [adjusted odds-ratio (OR) 3.93, p<0.001 and 5.98, p<0.001, respectively] and 3-year risk for incidence of MACE [adjusted Hazard ratio (HR) 1.98, p=0.025 and 2.40, p=0.01, respectively], and remained significant after adjusting for ADMA. The inventors therefore conclude that GABR may serve as a more comprehensive concept of reduced NO synthetic capacity compared to systemic arginine levels. Diminished GABR and high citrulline levels are associated with both development of atherosclerotic CAD and heightened risk for incident major adverse cardiac events.

### INTRODUCTION

Nitric oxide (nitrogen monoxide, NO) is an important endothelium-derived vasoactive substance critical to vascular health and homeostasis (Pacher et al., Physiol Rev, 87, p. 315-424 (2007)). Arginine serves as the sole nitrogen source for NO synthases. Nitric oxide is synthesized from arginine in a multistep reaction carried out by NO synthases, producing NO and citrulline (Figure 1). Previous studies indicate that diminished NO bioavailability is a critical predisposing factor in development of atherosclerotic heart disease (Tousoulis et al., Nat Clin Pract Cardiovasc Med, 4, p. 274-83 (2007)). Experimental animal model and preliminary clinical studies both indicate that intra-arterial or intravenous infusion of arginine improves NO production in the coronary arteries. See Boger et al., Circulation, 96, p. 1282-90 (1997), Drexler et al., Circulation, 89, p. 1615-23 (1994), Wang et al., J Am Coll Cardiol 23, p. 452-8 (1994), and Quyyumi et al., J Am Coll Cardiol, 30, p. 1220-7 (1997). However, results of oral arginine supplementation studies have been variable. Preli et al., Atherosclerosis, 162, p. 1-15 (2002), Schulman et al., JAMA, 295, p. 58-64 (2006), and Shirakiet et al., J Cardiol, 44, p. 13-20 (2004). Assessing arginine bioavailability may therefore provide an important insight into cardiovascular health not achievable by directly assessing arginine levels (Okyay et al., Coron Artery Dis, 18, p. 539-44 (2007)), and may potentially identify patients who may receive the greatest benefit from enhancing arginine bioavailability.

Arginine is the common substrate for both NO synthases and arginases (Figure 1). Huynh et al., Clin Exp Pharmacol Physiol, 33, p. 1-8 (2006). In several disease states arginase levels are increased and have been proposed to limit NO formation through increased arginine consumption. This limiting of NO formation results in indirect competition with NO synthases, the formation of ornithine, and subsequently citrulline. Morris et al., Am J Respir Crit Care Med, 170, p. 148-53 (2004) and Moss et al., Clin Sci (Lond), 107, p. 391-7 (2004). In turn, citrulline (also a direct product of NO synthases) may convert back to arginine via the formation of argininosuccinate (primarily in the kidneys). Morris SM, Jr., Vase Med, 10 Suppl 1, p. S83-7 (2005). Hence, it was hypothesized that the "bioavailability" of arginine may be impacted by different processes that may reduce arginine availability to NO synthases. The concept of "global arginine bioavailability ratio" (GABR) was therefore proposed to account for levels of the substrate (arginine) and its major catabolic products (ornithine and citrulline) *in vivo*. GABR is defined as the ratio arginine/(ornithine + citrulline).

Specifically, the hypothesis that plasma levels of GABR would be more highly predictive of the development and progression of cardiovascular diseases than plasma levels of free arginine was tested. This prediction was verified by measuring the prevalence of significant CAD at baseline and the subsequent incidence of MACE in their study population. The relative prognostic value of GABR with asymmetric dimethylarginine (ADMA), an endogenous direct NO synthases inhibitor; was also further explored.

### METHODS

***Study population.*** The Cleveland Clinic GENEBANK study is a large, single-center, prospective, cohort study with plasma, serum, and DNA repository obtained from sequential subjects undergoing elective diagnostic cardiac catheterization. The Cleveland Clinic Institutional Review Board approved the study, and all participants gave written informed consent. Clinical and demographic information was collected at the time of enrollment, and data were de-identified before analysis. The study utilized plasma samples obtained from 1,010 consecutive subjects enrolled in GENEBANK. The Framingham Risk score (including diabetes) was calculated for each subject, and the glomerular filtration rate was estimated by the Modification of Diet in Renal Disease (MDRD) formula. High sensitivity C-reactive protein (hsCRP), creatinine, fasting blood glucose and lipid profiles were measured on the Abbott ARCHITECT platform (Abbott Diagnostics, Abbott Park IL). Over the 3 years following enrollment, adjudicated outcomes were ascertained for all subjects.

***Data extraction and endpoints*.** Coronary artery disease (CAD) was defined as any clinical history of myocardial infarction (MI), percutaneous coronary intervention, coronary artery bypass surgery, acute coronary syndrome or angiographic evidence of CAD (>50% stenosis) in one or more major coronary arteries. A major adverse cardiovascular event (MACE) was defined as the occurrence of non-fatal myocardial infarction, non-fatal stoke, or death within 3 years of follow-up. All clinical outcomes were adjudicated with source documentation.

***Sample collection and measurements.*** Samples were collected from fasting subjects on the day of elective cardiac catheterization. Plasma aliquots analyzed were isolated from whole blood collected into EDTA (lavender top) tubes which were maintained at 0-4°C immediately following phlebotomy, processed within 4 hours of blood draw, and stored at -80°C until use. Plasma arginine, ornithine, citrulline and ADMA levels were determined by stable isotope dilution HPLC with on-line electrospray ionization tandem mass spectrometry (LC/ESI/MS/MS) using an API 365 triple quadruple mass spectrometer (Applied Biosystems, Foster, CA) with Ionics 10+ redesigned source as upgrade, and interfaced with a Cohesive HPLC (Franklin, MA). ¹³C₆-Arginine was used as the internal standard and added at the time of plasma aliquot thawing. Plasma proteins were precipitated with 80% methanol and supernatant, after centrifugation at 5,000 g for 10 min, 0°C, and were collected for determination of the analyte concentration as described (15).

***Statistical analysis.*** The Wilcoxon-Rank sum test for continuous variables and chi-square test for categorical variables were used to examine the difference between the groups. GABR was divided into quartiles for analysis. Unadjusted trends for increasing CAD, MACE at 3 years with increasing or decreasing quartiles were evaluated with the log-rank test of trend. Since diminished GABR (*i.e.*, lower values) portend greater risk, logistic regression models were developed to calculate odds ratios (ORs) or hazard ratios (HRs) associated with the first, second, and third quartiles of GABR, which were compared with the highest quartile. Adjustments were made for Framingham Global Risk Score, creatinine clearance, and plasma hsCRP levels. Kaplan-Meier analysis with Cox proportional hazards regression was used for time-to-event analysis. A Spearman correlation coefficient was calculated to summarize the correlation between GABR and ADMA. All analyses were performed using SAS version 8.2 (Cary, NC). P values <0.05 were considered statistically significant.

### RESULTS

***Study Population.*** Table 1 illustrates the clinical characteristics of the study population, stratified according to the presence or absence of CAD. As expected, patients with CAD were more likely to be older, and have one or more cardiovascular risk factors (diabetes mellitus and hypertension). In the analysis, plasma arginine level was normally distributed, whereas ornithine, citrulline, and GABR were not.

***Plasma levels of arginine***, ***GABR and CAD***. Compared to those without CAD, patients with CAD had significantly lower levels of plasma arginine, but higher levels of ornithine and citrulline (Table 1). This directly leads to significantly lower median GABR in patients with versus those without CAD [median (interquartile range, IQR): 1.06(0.75-1.31) versus 1.27(0.96-1.73), p <0.001]. After adjusting for traditional risk factors, creatinine clearance, and hsCRP, GABR levels remained significantly associated with prevalent CAD [Quartile 1 compared to Quartile 4; adjusted OR(95% confidence interval, CI): 3.93(2.55-6.05) p<0.001; Table 2]. Higher ornithine and citrulline levels were also associated with higher prevalence of CAD (Table 3). In contrast, while lower plasma arginine levels were associated with higher CAD prevalence across quartiles in unadjusted analysis, low plasma arginine levels were no longer a predictor of prevalence of CAD after adjusting for traditional risk factors, creatinine clearance, and hsCRP [adjusted OR (95%CI): 1.19(0.79-1.80), p=0.40; Table 2].

**Table 1. Baseline Characteristics of Study Population**

| | **No CAD** (n=4.02) | **CAD** (n=608) | **p**-**value** |
|---|---|---|---|
| **Demographics and Cardiovascular Risk Factors** | | | |
| Age (years) | 61 ± 8 | 66 ± 10 | <0.001 |
| Female (%) | 53% | 52% | 0.772 |
| Diabetes (%) | 15% | 45% | <0.001 |
| Hypertension (%) | 54% | 78% | <0.001 |
| Framingham Risk Score (mean ± SD) | 6.6 ± 3.4 | 8.5 ± 4.0 | <0.001 |
| Current Smokers (%) | 5% | 6% | 0.318 |

| **Laboratory Data** | | | |
|---|---|---|---|
| LDL cholesterol (mean ± SD) | 108 ± 33 | 100 ± 35 | <0.001 |
| HDL cholesterol (mean ± SD) | 53 ± 17 | 44 ± 14 | <0.001 |
| Triglycerides (median/IQR) | 117 (83, 168) | 141 (105, 212) | <0.001 |
| hsCRP, mg/dL (median/IQR) | 2.2 (1.1, 4.6) | 3.3 (1.7, 7.6) | <0.001 |
| Creatinine clearance (mean ± SD) | 99.2 ± 32.8 | 88.2 ± 40.0 | <0.001 |
| Arginine (µM) (mean ± SD) | 74 ± 24 | 68 ± 20 | <0.001 |
| Ornithine (µM) (median/IQR) | 35.9 (25.9, 49.1) | 41.3 (29.9, 56.6) | <0.001 |
| Citrulline (µM) (median/IQR) | 17.7 (13.7, 26.0) | 23.4 (17.5, 31.0) | <0.001 |
| GABR (median/IQR) | 1.27 (0.96, 1.73) | 1.06 (0.75, 1.31) | <0.001 |

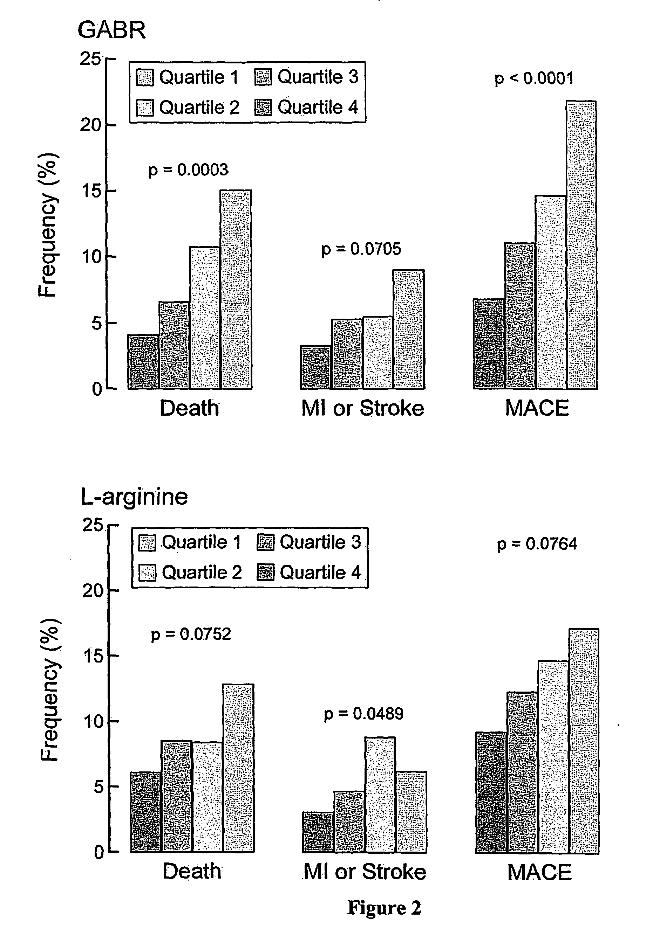

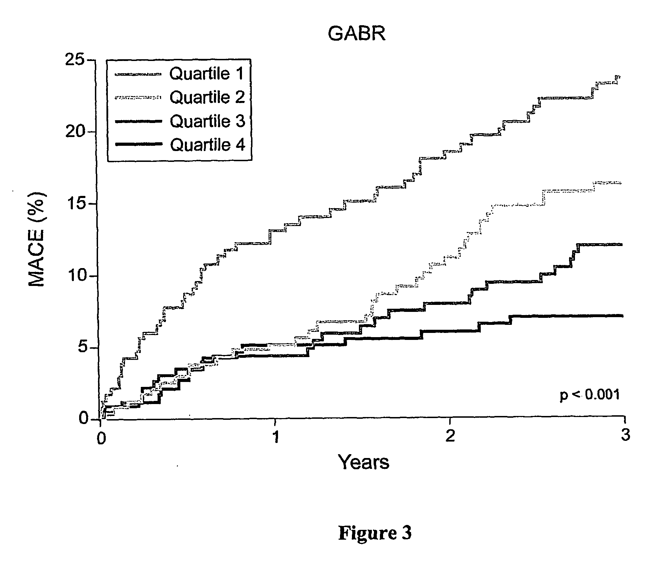

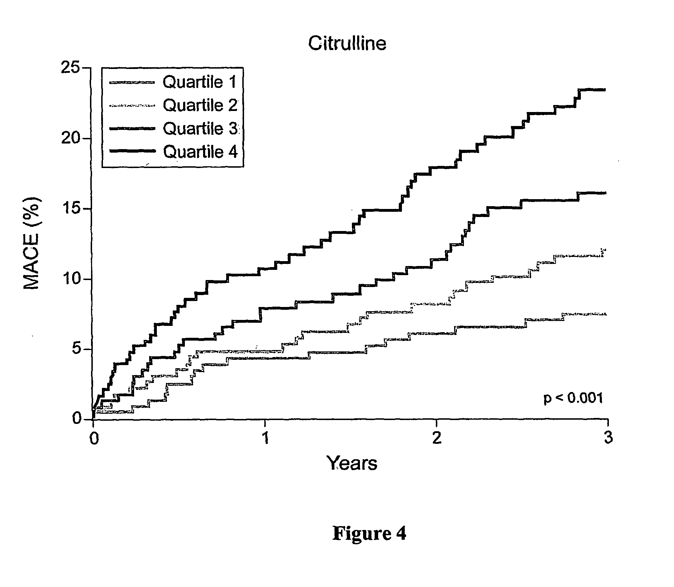

***Plasma arginine metabolite levels, GABR and MACE.*** Among the 991 subjects with 3-year follow-up, there were 126 (12.7%) incident MACE events. Analytes were again divided into quartiles for analyses of risk prediction, and the rates of non-fatal MI or stroke, all-cause mortality, as well as the composite MACE examined (Figure 2). Overall, GABR across decreasing quartiles provided consistent incremental association with increasing incident MACE. Figures 3 and 4 show the Kaplan-Meier time-to-event curves for the occurrence of MACE for each quartile of GABR and citrulline, respectively. After adjusting for Framingham Risk Score, hsCRP, and creatinine clearance, subjects with lowest absolute values of GABR and highest levels of citrulline showed the greatest risk for incident MACE over the three years after angiography. In contrast, arginine and ornithine quartiles did not predict incident MACE risk at 3 years (Tables 2 and 3).

**Table 2. Odds Ratio (Unadjusted and Adjusted) for Coronary Artery Disease (CAD) Prevalence and Major Adverse Cardiac Events (MACE) at 3 Years with Decreasing Quartiles of GABR and Arginine Levels**

| | **4^{th}** | **3^{rd}** | **2^{nd}** | **1^{st}** |
|---|---|---|---|---|
| | **quartile** | **Quartile** | **quartile** | **quartile** |
| **GABR** | **>1.46** | **1.14-1.46** | **0.82-1.14** | **<0.82** |
| **CAD Prevalence** | | | | |
| Unadjusted OR (95% CI) | 1.0 | 2.41 (1.68-3.47)* | 3.28 (2.26-4.75)* | 3.63 (2.50-5.28)* |
| Adjusted OR (95% CI) | 1.0 | 2.76 (1.86-4.09)* | 3.77 (2.49-5.72)* | 3.93 (2.55-6.05)* |
| **MACE at 3 years** | | | | |
| Unadjusted HR (95% CI) | 1.0 | 1.67 (0.89-3.12) | 2.27 (1.25-4.11)# | 3.64 (2.08-6.39)* |
| Adjusted HR (95% CI) | 1.0 | 1.41 (0.75-2.67) | 1.42 (0.77-2.63) | 1.98 (1.09-3.59)† |
| **Arginine (µM)** | **>82.8** | **68.5-82.8** | **55.3-68.4** | **<55.3** |
| **CAD Prevalence** | | | | |
| Unadjusted OR (95% CI) | 1.0 | 1.69 (1.18-2.42)# | 1.74 (1.21-2.49)# | 1.94 (1.35-2.79)* |
| Adjusted OR (95% CI) | 1.0 | 1.69 (1.12- 2.56)† | 1.23 (0.82-1.85) | 1.19 (0.79-1.80) |
| **MACE at 3 years** | | | | |
| Unadjusted HR (95% CI) | 1.0 | 1.32 (0.75-2.32) | 1.55 (0.90-2.66) | 1.85 (1.10-3.14)† |
| Adjusted HR (95% CI) | 1.0 | 1.17 (0.65-2.10) | 1.15 (0.66-2.01) | 1.22 (0.70-2.12) |

| | | | | |
|---|---|---|---|---|
| * p<0.001, # p<0.01, † p<0.05 | | | | |

Additional analyses were performed looking at the relationship between GABR and ADMA in predicting prevalence of CAD and incidence of MACE (3 year) within the cohort. Overall, GABR and ADMA showed a modest negative correlation (r =-0.24, p <0.001). After adjusting for ADMA, the lowest quartile of GABR remained independently predictive of both higher prevalence of CAD [adjusted OR (95% CI): 3.28(2.24-4.82), p<0.001] and higher incidence of MACE [adjusted HR (95% CI): 2.93(1.66-5.20), p<0.001] when compared to the highest quartile of GABR. Similar observations were made in the highest quartile of citrulline after adjusting for ADMA for higher incidence of MACE [adjusted HR(95% CI): 2.03(1.44-2.87), p<0.001].

**Table 3. Odds Ratio (Unadjusted and Adjusted) for Coronary Artery Disease (CAD) Prevalence and Major Adverse Cardiac Events (MACE) at 3 Years with Increasing Quartiles of Citrulline and Ornithine Levels**

| | **1st** | **2^{nd}** | **3^{rd}** | **4^{th}** |
|---|---|---|---|---|
| | **quartile** | **Quartile** | **quartile** | **quartile** |
| **Citrulline (µM)** | **<15.3** | **15.3-21.2** | **21.3-29.4** | **>29.4** |
| **CAD Prevalence** | | | | |
| Unadjusted OR (95% CI) | 1.0 | 1.50 (1.05-2.15)t | 2.91 (2.01-4.20)* | 3.17 (2.18-4.61)* |
| Adjusted OR (95% CI) | 1.0 | 1.67 (1.14-2.46)# | 3.63 (2.40-5.51)* | 5.98 (3.72-9.60)* |
| **MACE at 3 years** | | | | |
| Unadjusted HR (95% CI) | 1.0 | 1.61 (0.87-2.97) | 2.21 (1.2-3.96)# | 3.35 (1.93-5.82)* |
| Adjusted HR (95% CI) | 1.0 | 1.62 (0.87-3.01) | 1.89 (1.05-3.1) † | 2.40 (1.34-4.30)# |
| **Ornithine (µM)** | **<28.3** | **28.3-38.9** | **38.9-53.7** | **>53.8** |
| **CAD Prevalence** | | | | |
| Unadjusted OR (95% CI) | 1.0 | 1.30 (0.91-1.85) | 1.67 (1.17-2.39)# | 2.29 (1.58-3.32)* |
| Adjusted OR (95% CI) | 1.0 | 1.35 (0.92-1.99) | 2.32 (1.54-3.49)* | 3.95 (2.53-6.18)* |
| **MACE at 3 years** | | | | |
| Unadjusted HR (95% CI) | 1.0 | 1.12 (0.66-1.89) | 1.44 (0.86-2.40) | 1.54 (0.93-2.54) |
| Adjusted HR (95% CI) | 1.0 | 1.06 (0.62-1.83) | 1.16 (0.68-1.98) | 1.23 (0.73-2.06) |

| | | | | |
|---|---|---|---|---|
| * p<0.001, # p<0.01, † p<0.05 | | | | |

### DISCUSSION

The relationship between plasma arginine levels, NO biosynthesis and cardiovascular disease is complex. Arginine participates in various metabolic reactions that differ by compartments and are modulated by diet, cytokines, and hormones. It is the inventors understanding that this study is the largest one to date to address the clinical significance of assessing systemic levels of arginine metabolites for cardiovascular risks in humans, and the first to demonstrate the relationship between GABR and, independently, the relationship between citrulline levels and cardiovascular disease prevalence and future risk for major adverse cardiac events. These findings were independent of traditional cardiovascular risk factors, renal function, markers of inflammation, and even when accounting for levels of the endogenous NO synthase inhibitor ADMA.

Increasing arginine as a substrate for NO synthases has been postulated to increase in endothelial NO production. However, arginine supplementation studies have demonstrated mixed outcomes with respect to cardiovascular disease outcomes. The underlying mechanisms for this are not clear. Under physiologic conditions, the intracellular NO synthases should be adequately saturated with its arginine substrates. Enhanced NO production observed in response to elevations in extracellular arginine, despite high intracellular arginine concentrations, is often referred to as the "arginine paradox." Bode-Boger et al., Pharmacol Ther, 114, p. 295-306 (2007) and Tentolouris et al.,. Circulation, 110, p. e71 (2004). Several investigators have postulated that endogenous inhibitors of NO synthases, methylated arginine metabolites such as ADMA, may provide the explanation. Indeed, the inventors and others have demonstrated the clinical significance of elevated plasma ADMA levels in cardiovascular risks (15,18-20). Nicholls SJ, Wang Z, Koeth R, et al. Metabolic profiling of arginine and nitric oxide pathways predicts hemodynamic abnormalities and mortality in patients with cardiogenic shock after acute myocardial infarction. Circulation 2007;116:2315-24, Boger et al., Circulation, 98, p. 1842-7 (1998). Schulze et al., Am Heart J, 152, p. 493 e1-8 (2006), and Tang et al., Eur Heart J, 29, p. 2506-2531 (2008). In particular, data have suggested that a reduction in intracellular concentrations of competitive and non-competitive inhibitors by extracellular arginine displacement may augment NO synthase activity, thereby increasing NO production. Kielstein et al., Circulation, 109, p. 172-7 (2004).

The current data provide an alternative (or complementary) explanation to the arginine paradox, whereby a *relative* deficiency of arginine for NO synthases caused by augmented "catabolic pathways" of arginine (such as arginases) can in part contribute to underlying CAD and development of cardiovascular events. While *in vitro* studies have indicated an association between decreases in arginine concentrations, both systemic and locally, within atherosclerotic plaques, as well as impairment of the endothelial NO synthase-mediated stress responses (Suschek et al., Circulation, 107, p. 2607-14 (2003)), systemic arginine levels have not been correlated with prevalence of CAD. Okyay et al., Coron Artery Dis, 18, p. 539-44 (2007) Also, arginine supplementation studies have thus far failed to demonstrate improvement in cardiovascular outcomes. Schulman et al., JAMA, 295, p. 58-64 (2006) Several studies have suggested a relative arginine deficiency exists in subjects with hypertension or heart failure as a result of abnormal transport mechanisms across vascular cellular membranes. Kaye et al., Circulation, 102, p. 2707-12 (2000). Schlaich et al., Circulation, 110, p. 3680-6 (2004).

Extracellular concentrations of arginine can also depend on catabolism of the amino acid via several pathways including augmented degradation of arginine, which may lead to relative NO deficiency and subsequent progression of cardiovascular disease.

Arginases, in particular, have been shown to be upregulated in several disease states with components of inflammation including CAD, cystic fibrosis, sickle cell disease, and asthma. Durante et al., Clin Exp Pharmacol Physiol, 34, p. 906-11 (2007) Indeed, arginases are subject to regulation by inflammatory cytokines as well as inter-regulation by the arginine metabolites themselves. Satriano J., Amino Acids, 26, p. 321-9 (2004). Increased arginase activity in endothelial cells has been argued to promote a pro-atherogenic effect because of direct reduction of endothelial cell NO production via the conversion of arginine to ornithine. Indeed, it has been suggested that indirect strategies for elevating arginine (such as the inhibition of arginase) could prove more effective at improving intracellular arginine bioavailability than exogenous arginine administration. Further support for this approach may be seen in smooth muscle cells, where heightened arginine catabolic processes could result in increased production of collagen and enhanced cell proliferation attributable to the metabolism of ornithine into proline and polyamines, respectively. The recently demonstrated association between the arginase I gene polymorphism, rs2781666, and both heightened MI risk and increased carotid intimal medial thickness is consistent with the hypothesis that arginine catabolic processes are linked to CAD pathogenesis. Dumont et al., J Med Genet, 44, p. 526-31 (2007). Meanwhile, the downstream effects of diminished arginine bioavailability leading to progression of cardiovascular disease have also been investigated. One suggestion has been the impact of this decreased availability of arginine on the synthesis of NO by interfering with NO synthase mRNA translation via altered phosphorylation and activity of eukaryotic initiation factor. Lee et al., Proc Natl Acad Sci USA, 100, 4843-8 (2003).

### EXAMPLE 2

Plasma levels of citrulline can be shown to be correlated with the severity of heart failure. Blood samples from patients with known diagnoses of chronic heart failure (HF) and stable conditions, and patients with acute heart failure are obtained..

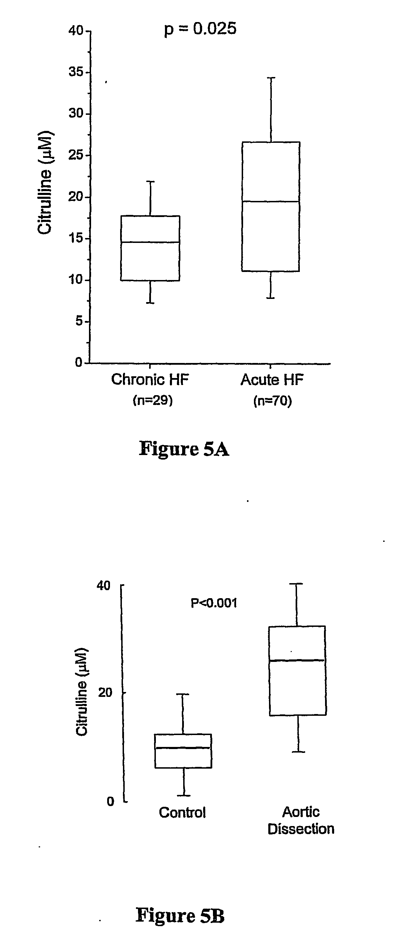

Samples are collected using ethylenediamenetetraacetic acid-plasma Vacutainers (Becton Dickinson and Company, Franklin Lakes, New Jersey), processed, and frozen at -80 °C until analyzed. Plasma citrulline levels are determined as described in Example 1. The results show increasing levels of citrulline levels are associated with a higher degree of HF. As shown in Figure 5A, the mean plasma citrulline levels in the acute HF cohort are significantly elevated compared with those of patients with chronic HF.

### EXAMPLE 3

Plasma levels of citrulline can be shown to have prognostic value in evaluating patients with chronic heart failure. The primary objective of this study is to determine the relationship between plasma citrulline levels and cardiac structure, systolic and diastolic performance, and overall prognosis in patients with chronic systolic HF.

The neurohormonal sub-study of the ADEPT (assessment of Doppler Echocardiography in Prognosis and Therapy) study has been previously described. See Troughton et al., J. Am. Coll. Cardiol. 43, 416-22 (2004). Patients with stable, chronic systolic heart failure (HF) (Left ventricular ejection fraction [LVEF] ≤ 35%, New York Heart Association functional class II to IV) undergo echocardiographic evaluation of systolic and diastolic performance as well as plasma sample collection. Clinical events (all-cause mortality, cardiac transplantation, or HF hospitalization) are tracked by scheduled telephone follow-up and validated by chart review as described by Troughton et al. (Am. J. Cardiol 96, 257-62 (2005)). Creatinine clearance may be calculated using the Crockcroft-Gault equation based on creatinine, age, and weight. Plasma B-type natriuretic peptide (BNP) levels may be analyzed by the Christchurch BNP assay.

Samples are collected using ethylenediaminetetraacetic acid plasma tubes, processed and frozen at -80°C until analyzed. Plasma citrulline levels are determined as described in Example 1. Laboratory analyses should be performed with investigators blind to the clinical outcomes.

Comprehensive transthoracic echocardiography can be performed using commercially available HDI 5000 (Phillips Medical Systems, Bothell, Washington) and Acuson Sequoia (Siemens Medical Solutions USA, Inc., Malvern, Pennsylvania) machines. Two-dimensional and color Doppler imaging can be performed in standard parasternal and apical views. Diastolic indexes (including pulse-wave Doppler, color M-mode, and tissue Doppler imaging) are acquired over 10 consecutive beats using sweep speeds of 50 cm/s and 100 cm/s using previously described techniques. Classification of diastolic stage is determined as follows: Stage I (impaired relaxation) consists of mitral E/A <1, deceleration time >220 ms, pulmonary vein S/D >1, atrial reversal (AR) <35 cm/s; Stage II (pseudonormal) shows mitral E/A = 1 to 2, pulmonary vein S/D <1, deceleration time <220 ms, AR ?35 cm/s; Stage III (restrictive) gives mitral E/Vp ratios. The LVEF and cardiac volumes are measured using the Simpson biplane method. Measurements are averaged over 3 cycles (5 cycles for atrial fibrillation), and 2 experienced individuals who are blind to the neurohormonal data make all measurements.

Plasma citrulline levels may be non-normally distributed and treated as nonparametric variables (expressed as median and interquartile range [IQR]). Analysis of variance or the Kruskal-Wallis test is used to assess differences in continuous clinical variables across citrulline tertiles according to whether or not the distribution is normal, whereas contingency table analysis is performed to assess differences in clinical proportions across citrulline tertiles. Normality is assessed by the Shapiro-Wilk W test. The Spearman rank correlation method is used as a nonparametric measure of association for correlations between plasma citrulline levels and all clinical variables. The odds ratios of having altered systolic or diastolic performances are calculated from multivariate logistic regression across 1st, 2nd, and 3rd tertiles of citrulline with respect to the 1st tertile (odds ratio = 1.0). Adjustments can be made for age and BNP levels. Kaplan-Meier survival plots can be calculated from baseline to time of all-cause mortality, cardiac transplantation, or HF hospitalization over a mean follow-up of 33 months. All univariate and multivariate Cox proportionality hazard analyses are also calculated with all-cause mortality, cardiac transplantation, or HF hospitalization as outcome, and with plasma citrulline levels treated as a categorical variable modeling differences in outcomes for patients within the highest 2 tertiles relative to the lowest of plasma citrulline. Receiver-operator characteristic curve analysis can be performed to determine the incremental prognostic value of citrulline with BNP. A p value <0.05 is considered statistically significant. Statistical analyses are performed using SAS version 9.1 and JMP version 5.1 (SAS Institute Inc., Cary, North Carolina).

It is expected that increasing plasma citrulline levels will be associated with a higher proportion of right ventricular systolic dysfunction. In multivariable stepwise logistic regression analysis using variables that show statistically significant correlation with logarithmic transformed plasma citrulline levels, only tissue Doppler imaging-derived septal Aa wave is expected to show an independent association with citrulline levels.

Patients with an elevated level of citrulline can be expected to experience a cardiovascular event, death or cardiac transplantation.

### EXAMPLE 4

Citrulline levels in healthy elderly subjects can also be used to predict risk for developing heart failure. The goal of the present study is to assess the risk for incident HF; therefore, patients will be excluded if they had prevalent HF, prevalent myocardial infarction (MI), prevalent stroke, or died prior to the initial visit. Follow-up for events will continue. Factors assessed during the initial exam may include age, race, gender, diabetes, hypertension, smoking, sub-clinical vascular disease, alcohol use, current medications, height, weight, blood pressure and laboratory measurements (citrulline and lipids) and are included as covariates in multivariable analysis. For the diagnosis of prevalent heart failure, self-reports are confirmed by components of a physical exam, or if necessary, by a validation protocol that includes surveys of treating physicians or review of medical records. For the diagnosis of incident heart failure, a physician's diagnosis of heart failure will be followed by a review of the participant's medical records. The incidence of heart failure is then determined by a committee based on diagnosis from a physician, as well as consideration of symptoms, signs, chest X-ray findings, and treatment of heart failure (current prescription for diuretic agent and either digitalis or vasodilator). Systolic and diastolic blood pressures are calculated from the mean of two consecutive readings in the seated position. Smoking is defined as current versus not. Low-density lipoprotein (LDL) cholesterol can be calculated by the Friedewald formula. Subclinical cardiovascular diseases (CVD) documented may include abnormalities in carotid intima-media thickness as monitored by ultrasound, ankle-arm index, elevated left ventricular mass by electrocardiography, and major electrocardiographic abnormalities. Follow-up interviews for events (including MI and stroke) may occur every 6 months and in person annually.

Citrulline is measured from frozen samples collected at the initial exam. Plasma citrulline levels are determined using the method described in Example 1.

A pre-specified statistical analysis plan is performed including quartile based analysis and comparisons made of the distribution of demographic characteristics and traditional cardiovascular risk factors across the quartile groups;. Differences in baseline characteristics are compared using the Cochrane-Armitage trend test for continuous variables and Chi Square tests for categorical variables. The association between citrulline and HF is determined with multivariate Cox proportional hazards regression models. To evaluate the contribution of citrulline quartiles as a marker of risk, models are generated in stages: unadjusted; then adjusted for demographics and cardiovascular risk factors. Analysis is performed with a time-dependent variable for incident MI added to the model to evaluate the effect of controlling for this intervening event on the association of baseline characteristics censoring for participants with incident MI.

It is expected that higher levels of citrulline will be associated with higher incident HF.

The inventors have developed two multivariate models which can be used to explore the interaction between citrulline levels in blood and traditional cardiac risk factors and myocardial infarction. In model 1, the inventors treat MI as a time-dependent covariate. After adjusting for age,. gender, systolic blood pressure (SBP), smoking, low-density lipoprotein (LDL) cholesterol, diabetes mellitus, any subclinical cardiovascular disease and MI as a time-dependent covariate, it is expected that elevated citrulline levels will predict subjects at increased risk for developing incident HF.

### EXAMPLE 5

Citrulline levels in patients can also be used to predict risk for developing aortic disorders such as aortic dissection or aortic aneurysms, or to distinguish between healthy patients and those having an aortic disorder.. For predictive studies, patients are excluded if they previously exhibited an aortic disorder such as aortic dissection or aortic aneurysm. Other factors that can be assessed during the initial exam include age, race, gender, diabetes, hypertension, smoking, sub-clinical vascular disease, alcohol use, current medications, height, weight, blood pressure and laboratory measurements (citrulline and lipids) and are included as covariates in multivariable analysis. For the diagnosis of an aortic disorder such as aortic dissection or aortic aneurysm, reports are confirmed by a physical exam or a review of medical records. Preferably the aortic disorder is confirmed using diagnostic imaging technology. Follow-up interviews or examination for aortic disorders (including aortic dissection and/or aortic aneurysm) may occur every 3 months, every 6 months, annually, or using other schedules. The results of such an analysis are shown in Figure 5B, which shows a much higher level of citrulline in subjects diagnosed with aortic dissection as compared with control subjects.

To correlate citrulline levels with the development of aortic disorders, the level of citrulline is first measured from frozen samples collected at the initial exam. Plasma citrulline levels are determined using the method described in Example 1. Patients are then evaluated at 3 month, 6 month, 1 year, or another interval such as 2 or 3 years to identify the number of patients who have experienced or are experiencing an aortic disorder. The number of aortic dissections and aortic aneurysms may be separately tabulated.

A pre-specified statistical analysis plan is performed including quartile based analysis and comparisons made of the distribution of demographic characteristics and traditional cardiovascular risk factors across the quartile groups;. Differences in baseline characteristics are compared using the Cochrane-Armitage trend test for continuous variables and Chi Square tests for categorical variables. The association between citrulline and aortic disorders is determined with multivariate Cox proportional hazards regression models. To evaluate the contribution of citrulline quartiles as a marker of risk, models are generated in stages: unadjusted; then adjusted for demographics and cardiovascular risk factors.

The results demonstrated that patients with aortic dissection exhibit significantly higher levels of citrulline. It is also expected that higher levels of citrulline will be associated with higher incidence of aortic disorders such as aortic dissection and aortic aneurysm. In addition, multivariate models can be used to explore the interaction between citrulline levels in blood and traditional cardiac risk factors. In multivariate models, after adjusting for age,. gender, systolic blood pressure (SBP), smoking, low-density lipoprotein (LDL) cholesterol, diabetes mellitus, any subclinical cardiovascular disease and MI as a time-dependent covariate, it is expected that elevated citrulline levels will predict subjects at increased risk for developing aortic disorders such as aortic dissection and/or aortic aneurysm.

Although only a few exemplary embodiments have been described in detail, those skilled in the art will readily appreciate that many modifications are possible in the exemplary embodiments without materially departing from the novel teachings and advantages of this disclosure.

## Claims

1. A method for characterizing a human subject's risk of having or developing cardiovascular disease (CVD), or for monitoring a human subject's response to intervention aimed at reducing risk of CVD, wherein said method comprises a step of determining a level of free citrulline in a bodily sample of urine, blood, serum, or plasma from the subject, wherein the subject's level of free citrulline provides a risk predictor that is independent of arginine bioavailability ratios.

2. A method according to claim 1, which is a method for characterizing a human subject's risk of having or developing cardiovascular disease (CVD) comprising:
determining a level of free citrulline in a bodily sample of urine, blood, serum, or plasma, from the subject;
comparing the subject's level of free citrulline with a control value based on levels of free citrulline in comparable bodily samples from a control population; and
characterizing the subject's risk of having CVD based on said comparison;
wherein the subject is characterized as being at risk of having CVD if the subject's level of free citrulline is greater than the control value.

3. The method of claim 2, wherein the cardiovascular disease is heart failure, aortic dissection, aortic aneurysm, or a major adverse cardiac event.

4. The method of claim 2 or claim 3, wherein the control value is a single representative value or a range of representative values and is based upon the levels of free citrulline in comparable bodily samples from the general population or a select population of human subjects.

5. The method of claim 2 or claim 3, wherein the control value comprises ranges divided into a plurality of groups, and said comparing step comprises determining into which group the subject's level of said risk predictor falls.

6. The method of claim 5, wherein the plurality of groups comprises at least a high risk range and a low risk range.

7. The method of any one of claims 2 to 6, further comprising prescribing an intervention or lifestyle changes targeted at reducing the risk of CVD to a subject who has been characterized as being at risk of having CVD.

8. The method of any one of claims 2 to 6, wherein the subject's level of free citrulline is determined by an immunological technique, mass spectrometry, high performance liquid chromatography (HPLC), or any combination thereof.

9. A method according to claim 1, which is a method for monitoring a human subject's response to intervention aimed at reducing risk of CVD comprising:
determining a first and second level of free citrulline in a bodily sample of urine, blood, serum, or plasma, from the subject before and after administration of the intervention, respectively;
comparing the subject's first and second levels of free citrulline; and
characterizing the subject's response to the intervention based on said comparison;
wherein the subject is characterized as responding to the intervention if the subject's second level of free citrulline is lesser than the first level of free citrulline.

10. The method of claim 9, wherein the cardiovascular disease is heart failure, aortic dissection, aortic aneurysm, or a major adverse cardiac event.

11. The method of claim 9 or claim 10, wherein the intervention comprises administration of a therapeutic agent.

## Patentansprüche

1. Verfahren zur Charakterisierung des Risikos bei einem menschlichen Individuum, an einer Herz-Gefäß-Erkrankung (CVD) zu leiden oder diese zu entwickeln, oder zur Überwachung des Ansprechens eines menschlichen Individuums auf einen Eingriff mit dem Ziel, das Risiko einer CVD zu reduzieren, worin das Verfahren einen Schritt des Bestimmens des Ausmaßes an freiem Citrullin in einer Probe Urin, Blut Serum oder Plasma aus dem Körper des Individuums umfasst, worin das Ausmaß an freiem Citrullin bei dem Individuum eine Risikoprognose bereitstellt, die von Argininbioverfügbarkeitsverhältnissen unabhängig ist.

2. Verfahren nach Anspruch 1, das ein Verfahren zur Charakterisierung des Risikos bei einem menschlichen Individuum ist, an einer Herz-Gefäß-Erkrankung (CVD) zu leiden oder diese zu entwickeln, das Folgendes umfasst:
das Bestimmen eines Ausmaßes an freiem Citrullin in einer Probe Urin, Blut Serum oder Plasma aus dem Körper des Individuums;
das Vergleichen des Ausmaßes an freiem Citrullin bei dem Individuum mit einem Kontrollwert basierend auf Ausmaßen an freiem Citrullin in vergleichbaren Körperproben aus einer Kontrollpopulation; und
das Charakterisieren des Risikos bei dem Individuum, an einer CVD zu leiden, basierend auf dem Vergleich;
worin das Individuum als gefährdet, an einer CVD zu leiden, charakterisiert wird, wenn das Ausmaß an freiem Citrullin bei dem Individuum größer ist als der Kontrollwert.

3. Verfahren nach Anspruch 2, worin die Herz-Gefäß-Erkrankung Herzinsuffizienz, Aortendissektion, Aortenaneurysma oder ein größeres unerwünschtes Ereignis im Zusammenhang mit dem Herz ist.

4. Verfahren nach Anspruch 2 oder Anspruch 3, worin der Kontrollwert ein einzelner repräsentativer Wert oder ein Bereich von repräsentativen Werten ist und auf den Ausmaßen an freiem Citrullin in vergleichbaren Körperproben aus der allgemeinen Bevölkerung oder einer ausgewählten Population menschlicher Individuen basiert.

5. Verfahren nach Anspruch 2 oder Anspruch 3, worin der Kontrollwert in eine Vielzahl von Gruppen unterteilte Bereiche umfasst und der Vergleichsschritt das Bestimmen umfasst, in welche Gruppe der Wert der Risikoprognose bei dem Individuum fällt.

6. Verfahren nach Anspruch 5, worin die Vielzahl von Gruppen zumindest einen Bereich hohen Risikos und einen Bereich geringen Risikos umfasst.

7. Verfahren nach einem der Ansprüche 2 bis 6, das des Weiteren das Vorschreiben eines Eingriffs oder von Veränderungen in der Lebensführung an ein Individuum, das als gefährdet, an CVD zu leiden, charakterisiert wurde, mit dem Ziel, das Risiko einer CVD zu reduzieren, umfasst.

8. Verfahren nach einem der Ansprüche 2 bis 6, worin das Ausmaß an freiem Citrullin bei dem Individuum mittels eines immunologischen Verfahrens, Massenspektrometrie, Hochleistungsflüssigchromatographie (HPLC) oder einer beliebigen Kombination aus diesen bestimmt wird.

9. Verfahren nach Anspruch 1, das ein Verfahren zur Überwachung des Ansprechens eines menschlichen Individuums auf einen Eingriff mit dem Ziel ist, das Risiko einer CVD zu reduzieren, das Folgendes umfasst:
das Bestimmen eines ersten und eines zweiten Ausmaßes an freiem Citrullin in einer Probe Urin, Blut Serum oder Plasma aus dem Körper des Individuums vor bzw. nach der Verabreichung des Eingriffs;
das Vergleichen des ersten und des zweiten Ausmaßes an freiem Citrullin bei dem Individuum; und
das Charakterisieren des Ansprechens des Individuums auf den Eingriff basierend auf dem Vergleich;
worin das Individuum als auf den Eingriff ansprechend charakterisiert wird, wenn das zweite Ausmaß an freiem Citrullin bei dem Individuum geringer ist als das erste Ausmaß an freiem Citrullin.

10. Verfahren nach Anspruch 9, worin die Herz-Gefäß-Erkrankung Herzinsuffizienz, Aortendissektion, Aortenaneurysma oder ein größeres unerwünschtes Ereignis im Zusammenhang mit dem Herz ist.

11. Verfahren nach Anspruch 9 oder Anspruch 10, worin der Eingriff die Verabreichung eines therapeutischen Mittels umfasst.

## Revendications

1. Procédé de caractérisation d'un risque pour un sujet humain d'avoir ou de développer une maladie cardiovasculaire, ou de surveillance d'une réponse d'un sujet humain à une intervention ayant pour objectif de réduire le risque de maladie cardiovasculaire, ledit procédé comprenant une étape de détermination d'un niveau de citrulline libre dans un échantillon corporel d'urine, de sang, de sérum ou de plasma issu du sujet, dans lequel le niveau de citrulline libre du sujet fournit un prédicteur de risque qui est indépendant de rapports de biodisponibilité de l'arginine.

2. Procédé selon la revendication 1, qui est un procédé de caractérisation d'un risque pour un sujet humain d'avoir ou de développer une maladie cardiovasculaire comprenant :
la détermination d'un niveau de citrulline libre dans un échantillon corporel d'urine, de sang, de sérum ou de plasma issu du sujet ;
la comparaison du niveau de citrulline libre du sujet à une valeur témoin sur la base de niveaux de citrulline libre dans des échantillons corporels comparables issus d'une population témoin ; et
la caractérisation du risque pour un sujet d'avoir une maladie cardiovasculaire basée sur ladite comparaison ;
dans lequel le sujet est caractérisé comme présentant le risque d'avoir une maladie cardiovasculaire si le niveau de citrulline libre du sujet est supérieur à la valeur témoin.

3. Procédé selon la revendication 2, dans lequel la maladie cardiovasculaire est une insuffisance cardiaque, une dissection aortique, un anévrisme aortique ou un événement cardiaque indésirable majeur.

4. Procédé selon la revendication 2 ou la revendication 3, dans lequel la valeur témoin est une seule valeur représentative ou une plage de valeurs représentatives et est basée sur les niveaux de citrulline libre dans des échantillons corporels comparables issus de la population totale ou d'une population sélectionnée de sujets humains.

5. Procédé selon la revendication 2 ou la revendication 3, dans lequel la valeur témoin comprend des plages divisées en une pluralité de groupes, et ladite étape de comparaison comprend la détermination du groupe dans lequel se trouve le niveau dudit prédicteur de risque pour le sujet.

6. Procédé selon la revendication 5, dans lequel la pluralité de groupes comprend au moins une plage de risque élevée et une plage de risque faible.

7. Procédé selon l'une quelconque des revendications 2 à 6, comprenant en outre la prescription d'une intervention ou de changements de mode de vie dans l'objectif de réduire le risque de maladie cardiovasculaire chez un sujet qui a été caractérisé comme présentant un risque d'avoir une maladie cardiovasculaire.

8. Procédé selon l'une quelconque des revendications 2 à 6, dans lequel le niveau de citrulline libre du sujet est déterminé par une technique immunologique, une spectrométrie de masse, une chromatographie liquide haute performance (CLHP) ou toute combinaison de celles-ci.

9. Procédé selon la revendication 1, qui est un procédé de surveillance d'une réponse d'un sujet humain à une intervention ayant pour objectif de réduire le risque de maladie cardiovasculaire comprenant :
la détermination d'un premier et d'un second niveau de citrulline libre dans un échantillon corporel d'urine, de sang, de sérum ou de plasma, issu du sujet avant et après administration de l'intervention, respectivement ;
la comparaison des premier et second niveaux de citrulline libre du sujet ; et
la caractérisation de la réponse du sujet à l'intervention sur la base de ladite comparaison ;
dans lequel le sujet est caractérisé comme répondant à l'intervention si le second niveau de citrulline libre du sujet est inférieur au premier niveau de citrulline libre.

10. Procédé selon la revendication 9, dans lequel la maladie cardiovasculaire est une insuffisance cardiaque, une dissection aortique, un anévrisme aortique ou un événement cardiaque indésirable majeur.

11. Procédé selon la revendication 9 ou la revendication 10, dans lequel l'intervention comprend l'administration d'un agent thérapeutique.
